# EUROPEAN PATENT SPECIFICATION

(11) **EP 4 188 246 B1**
(45) Date of publication and mention of the grant of the patent: **25.09.2024**
(21) Application number: 21742420.9
(22) Date of filing: 14.07.2021
(51) Int. Cl.: A61B 17/221

(54) **AN ELONGATED DEVICE WITH AN IMPROVED ATTACHMENT OF ITS ELEMENTS FOR THE MOBILIZATION OF BLOOD CLOTS FOR THE EXTRACTION OF AN OCCLUSION FROM A BLOOD VESSEL**
EINE LÄNGLICHE VORRICHTUNG MIT EINER VERBESSERTEN BEFESTIGUNG IHRER ELEMENTE ZUR MOBILISIERUNG VON BLUTGERINNSELN ZUR EXTRAKTION EINES VERSCHLUSSES AUS EINEM BLUTGEFÄSS
UN DISPOSITIF ALLONGÉ AVEC UNE FIXATION AMÉLIORÉE DE SES ÉLÉMENTS POUR LE DEPLACEMENT DE CAILLOTS SANGUINS POUR L'EXTRACTION D'UNE OCCLUSION D'UN VAISSEAU SANGUIN

(30) Priority: 03.08.2020 EP 20382718; 03.08.2020 EP 20382719; 26.02.2021 EP 21382165
(43) Date of publication of application: 07.06.2023
(73) Proprietor: Anaconda BioMed, SL, 08192 Sant Quirze del Vallès Barcelona (ES)
(72) Inventor: GARCIA SABIDO, Daniel, 08720 Vilafranca del Penedès (ES); LIZARAZU GONZALEZ, Ane, 08013 Barcelona (ES); GALVE MURILLO, Iñaki, 08012 Barcelona (ES); ARAD HADAR, Ofir, 08197 Sant Cugat del Vallès (ES); JARA MUNS, Francesc, 08029 Barcelona (ES); PACE, Alizée, 1295 Mies, Vaud (CH); FERNÁNDEZ SÁNCHEZ, David, 08206 Sabadell (ES); NISSL, Thomas J.W., 37318 Mackenrode (DE)
(74) Representative: Torner, Juncosa I Associats, SL
(86) International application number: PCT/EP2021/069623
(87) International publication number: WO 2022/028833

(56) References cited:
- CN-A- 107 198 554
- US-A1- 2012 083 868
- US-A1- 2019 110 805

## Description

### Technical Field

The present invention is directed, in general, to the field of medical devices to restore blood flow or remove thrombus. In particular, the invention relates to an elongated device with an improved attachment of its elements. The elongated device is adapted to be maneuvered/manipulated during a medical intervention. The invention also relates to a method for connecting a working element of an elongated device to a pusher to enable the pusher to maneuver the working element.

### Background of the invention

Elongated medical devices for restoring blood flow or remove thrombus from a blood vessel during a thrombectomy intervention have to be advanced, pulled, and kept intact within the blood vessel during the intervention. Control and advancement of these devices are challenging as the elongated devices must undergo push, pull, and torque moves. Because of the length of these elongated devices and the movements and forces that the user must apply to the elongated device during the thrombectomy intervention, it is often difficult to maintain a secure attachment/connection of the elements that are part of the elongated device.

CN 107198554 B discloses an elongated medical device for restoring blood flow or remove thrombus from a blood vessel.

Current technology for blood flow restoration or thrombus removal often breaks or separates during use [1, 2], which may cause serious health consequences to the patients.

New elongated devices with an improved attachment of the elements/components thereof are therefore needed.

### References:

[1] Penumbra Inc. Recalls 3D Revascularization Device Due to Wire Material That May Break or Separate During Use. FDA recall. https://www.fda.gov/medical-devices/medical-device-recalls/penumbra-inc-recalls-3d-revascularization-device-due-wire-material-may-break-or-separate-during-use
[2] Unwanted detachment of the Solitaire device during mechanical thrombectomy in acute ischemic stroke. Journal of Neurolnterventional Surgery 2016 Dec;8(12):1226-1230. doi: 10.1136/neurintsurg-2015-012156.

### Description of the Invention

To overcome the drawbacks of the prior art, the present invention proposes, according to one aspect, an elongated device with an improved attachment of their elements. The elongated device can be advanced distally and withdrawn proximally from a proximal end and rotated about a longitudinal axis from the proximal end. The elongated device comprises a working element (e.g., a stent retriever or other medical device) comprising a first connection portion extending proximally, the first connection portion having a first attachment surface; a pusher comprising a second connection portion extending distally, the second connection portion having a second attachment surface facing and extending longitudinally aligned to the first attachment surface to define an overlapping portion and to define first and second seams extending longitudinally along first and second lateral extents of the overlapping portion; a first weld attaching the first and second connection portions, extending from the first seam toward the second seam (i.e. laterally); and a second weld attaching the first and second connection portions, extending from the second seam toward the first seam.

In an embodiment, a cross-sectional shape of the first connection portion perpendicular to the longitudinal axis is a section of an annulus. In another embodiment, a cross-sectional shape of the second connection portion perpendicular to the longitudinal axis is a circle.

In an embodiment, the first weld comprises a plurality of welding points along the first seam. In another embodiment, the second weld comprises a plurality of welding points along the second seam. In some embodiments, the plurality of welding points of the first and second welds are successive, i.e., the points are aligned.

In some embodiments, the first weld can further comprise a plurality of consecutive welding points. In some embodiments, the second weld can further comprise a plurality of consecutive welding points. Thus, a welding seam is provided on top of the welding points previously applied.

In an embodiment, the first weld and the second weld each extend along an entire length of the overlapping portion.

In an embodiment, glue can be added over the first and second welds.

In an embodiment, a ratio of a cross-sectional area of the first connection portion perpendicular to the longitudinal axis to a corresponding cross-sectional area of the second connection portion perpendicular to the longitudinal axis is in a range of 1:4 to 2:1.

In an embodiment, a radius of the second attachment surface is smaller than a radius of the first attachment surface.

In an embodiment, the elongated device also includes a jacket extending around at least part of the overlapping portion. The jacket can extend proximally from the overlapping portion around at least part of the pusher.

Additionally, the elongated device can also include a radiopaque element disposed at a proximal end of the overlapping portion. In an embodiment, the radiopaque element comprises a coil extending around at least part of the pusher.

In an embodiment, the elongated device also comprises a jacket extending around the radiopaque element. The jacket can extend around at least part of the overlapping portion.

Embodiments of the present invention also provide, according to another aspect, a method for connecting a working element to a pusher to enable the pusher to maneuver the working element (e.g. advance the working element distally, withdraw the working element proximally, and rotate the working element about a longitudinal axis from a proximal end of the pusher), the distal working element comprising a first connection portion extending proximally, the first connection portion having a first attachment surface, the pusher comprising a second connection portion extending distally, the second connection portion having a second attachment surface. The method comprises disposing the first attachment surface longitudinally aligned to the second attachment surface to form an overlapping portion and to form first and second seams extending parallel to the longitudinal axis along first and second lateral extents of the overlapping portion; forming a first weld extending from the first seam toward the second seam, and forming a second weld extending from the second seam toward the first seam.

In an embodiment, a cross-sectional shape of the first connection portion perpendicular to the longitudinal axis is a section of an annulus. In another embodiment, a cross-sectional shape of the second connection portion perpendicular to the longitudinal axis is a circle.

In an embodiment, the step of forming the first weld comprises forming a plurality of welding points extending from the first seam toward the second seam. In another embodiment, the step of forming the second weld comprises forming a plurality of welding points extending from the second seam toward the first seam. Particularly, the first and second welds are formed by sequentially forming the plurality of welding points (i.e. the welding points are aligned).

In an embodiment, the step of forming the first weld further comprises consecutively forming a plurality of welding points extending from the first seam toward the second seam. In another embodiment, the step of forming the second weld further comprises consecutively forming a plurality of welding points extending from the second seam toward the first seam.

When energy, e.g. laser energy, is applied to form the first and second welds a heat affected zone appears (i.e. accumulation of heat in a specific zone) which can provoke a breakage of the first, the second, or both connection portions. To avoid that breakage, in some embodiments, the step of forming the first weld further comprises forming a first welding point of the plurality of welding points at a distal portion of the first seam and forming each other welding point of the plurality of welding points at a more proximal location along the first seam than a prior formed welding point of the plurality of welding points. In some embodiments, the step of forming the second weld further comprises forming a first welding point of the plurality of welding points at a distal portion of the second seam and forming each other welding point of the plurality of welding points at a more proximal location along the second seam than a prior formed welding point of the plurality of welding points.

In some embodiments, the first weld can extend along an entire length of the first seam and the second weld can extend along an entire length of the second seam.

In an embodiment, the step of forming a first weld comprises forming a first welding point at a proximal end of the first seam and a second welding point between the proximal end and the distal end of the first seam before forming a welding point at any other points along the first seam.

In an embodiment, the step of forming the second weld comprises forming a first welding point at a proximal end of the second seam and a second welding point between the proximal end and the distal end of the second seam before forming a welding point at any other points along the second seam.

In an embodiment, the step of forming the first weld comprises directing energy laterally at the first seam toward the second seam. In an embodiment, the step of forming the second weld comprises directing energy laterally at the second seam toward the first seam.

In an embodiment, the method also comprises the step of adding glue over the first and second welds.

In an embodiment, the method also comprises the step of placing a radiopaque element at a proximal end of the overlapping portion. In an embodiment, the radiopaque element can comprise a coil extending around at least part of the pusher.

In an embodiment, the method also comprises the step of assembling, or placing, the radiopaque element onto a distal end of the pusher and pushing the radiopaque element towards a proximal end of the pusher.

In an embodiment, the method further comprises covering at least part of the overlapping portion with a jacket. In some embodiments, a portion of the pusher proximal to the overlapping portion can also be covered with the jacket.

In an embodiment, the method further comprises covering the radiopaque element with a jacket. In some embodiments, the jacket also extends around at least part of the overlapping portion.

In an embodiment, before inserting the jacket, the method further comprises placing, or pushing back, the radiopaque element to the distal end of the pusher (or at the proximal end of the overlapping portion), without covering the overlapping portion.

In yet another embodiment, the method comprises covering at least part of the overlapping portion with a jacket (or inner jacket), the jacket extending proximally from the overlapping portion around at least part of the pusher; placing a radiopaque element at a proximal end of the overlapping portion, the radiopaque element comprising a coil extending around at least part of the pusher; and covering the radiopaque element with a jacket (or outer jacket), the jacket extending around at least part of the overlapping portion.

In an embodiment, before inserting the inner jacket, the method further comprises assembling, or placing, the radiopaque element onto a distal end of the pusher and pushing the radiopaque element towards a proximal end of the pusher.

In another embodiment, before inserting the outer jacket, the method further comprises placing, or pushing back, the radiopaque element to the distal end of the pusher (or at the proximal end of the overlapping portion), without covering the overlapping portion.

In an embodiment, a ratio of a cross-sectional area of the first connection portion perpendicular to the longitudinal axis to a corresponding cross-sectional area of the second connection portion perpendicular to the longitudinal axis is in a range of 1:4 to 2:1.

In an embodiment, a radius of the second attachment surface is smaller than a radius of the first attachment surface.

In some embodiments, the working element of the elongated device comprises a working portion having a plurality of crowns (or rows) of cells. Each cell of the working portion includes an open area bordered by struts, where: a distal end of each cell in a first crown of the plurality of crowns is contiguous with a proximal end of a corresponding cell in a third crown of the plurality of crowns, a distal end of each cell in a second crown of the plurality of crowns is contiguous with a proximal end of a corresponding cell in a fourth crown of the plurality of crowns, the second crown is disposed distal to the first crown and proximal to the third crown, and the fourth crown is disposed distal to the third crown. Moreover, first and second opposite midportions of each cell in each of the first, second, third and fourth crowns each are contiguous with a midportion of an adjacent cell in such crown.

In some embodiments, the number of crowns of cells depends on the desired length of the working portion. In some embodiments, the working portion can have at least four crowns of cells.

In some embodiments, the working portion can have between four and twelve crowns of cells. Particularly, the working portion can have between six and ten crowns of cells. More in particular, the working portion can have seven, eight or nine crowns of cells.

In some embodiments, each crown of cells in the working portion can have at least three cells. In some embodiments, each crown of cells in the working portion can have at least four cells. In some embodiments, each crown of cells in the working portion can have between four and ten cells, particularly, between four and eight cells. In an embodiment, each crown of cells in the working portion can have four or six cells.

In an embodiment, each crown of cells in the working portion can have four cells. In another embodiment, each crown of cells in the working portion can have six cells.

In an embodiment, the working portion comprises at least four crowns of cells and each crown of cells comprises at least four cells. In another embodiment, the working portion comprises eight crowns of cells and each crown of cells comprises four cells. In another embodiment, the working portion comprises seven crowns of cells and each crown of cells comprises four cells. In another embodiment, the working portion comprises nine crowns of cells and each crown of cells comprises six cells.

In some embodiments, the working element also comprises a tapered portion extending proximally from the proximal end of the working portion, the tapered portion also having a plurality of struts. In an embodiment, the tapered portion has a smaller diameter at a proximal end than an expanded diameter of the working portion. In another embodiment, at least some of the tapered portion struts have a width greater than a width of the working portion struts.

In an embodiment, first and second tapered portion struts of the plurality of tapered portion struts converge from a proximal end of the working portion to a distal end of a proximal connection portion to partially define a proximal cell. In some embodiments, the elongated device comprises a pusher extending proximally from the proximal connection portion.

In an embodiment, a third tapered portion strut of the plurality of tapered portion struts extends distally from the first tapered portion strut from a point distal to the proximal connection portion. In another embodiment, a fourth tapered portion strut of the plurality of tapered portion struts extends distally from the second tapered portion strut from a point distal to the proximal connection portion, the third and fourth tapered struts partially defining the proximal cell.

In an embodiment, the third and fourth tapered portion struts converge at the proximal end of the working portion.

According to an embodiment, the elongated device is configured to have a compressed (or retracted) configuration (or position or state) with a compressed diameter, and an expanded configuration (or position or state) with an expanded diameter. The elongated device has the compressed configuration for example when it is located within a lumen of a narrow catheter or a lumen of a narrow vessel with a small diameter, and has the expanded configuration for example when it is located within a lumen of a vessel with large diameter. The elongated device is configured to self-expand from the compressed position (with a compressed diameter or first diameter) to an expanded position (with an expanded diameter or second diameter). In other words, the elongated device is self-expandable from the compressed configuration to the expanded configuration. Additionally, the elongated device is configured to adapt its shape to the surrounding blood vessel, thus the elongated device can have a first diameter in a narrow vessel with a small diameter and a second diameter in a wide vessel with a large diameter. Particularly, according to this embodiment, the elongated device, or the working element thereof, should be understood as a clot mobilizer device.

According to some embodiments of the invention, the working portion is configured to expand from a compressed diameter of less than 1.5 mm to an expanded diameter of at least 3.5 mm and to exert an outward radial force between 0.75 N and 3 N at every diameter between and including the compressed diameter and the expanded diameter.

In an embodiment, the working portion is configured to have a compressed diameter of less than 1.5 mm and to exert an outward radial force between 1.75 N and 3 N when the compressed diameter is around 1.5 mm. In another embodiment, the working portion is configured to have an expanded diameter of at least 3.5 mm and to exert an outward radial force between 0.75 N and 1.5 N when the expanded diameter is around 3.5 mm.

Advantageously, in the proposed elongated device the outward radial forces are maintained for a broad range of vessels, resulting in a flat curve behavior and a better pushability. This behavior allows lower radial forces in small diameters and larger radial forces in large diameters, thus without being out of a limited safety window between 1 N and 2.1 N. In an embodiment, the working portion is configured to exert an outward radial force in the range, inside the safety window, between 1 N and 2.1 N at every diameter between and including a compressed diameter of 1.5 mm and an expanded diameter of 3.5 mm. Remarkably, due to the fact of having less radial force in small diameters, the risk of damaging the vessel is reduced. Moreover, the proposed elongated device can be used in vessels of different diameters (between 1.5-4 mm).

In an embodiment, the crowns of cells of the elongated device define a tubular-shaped section forming a cylindrically closed structure. To that effect, the proposed elongated device can be manufactured by producing specified cuts either on a tube or on a wire. In another embodiment, the elongated device is manufactured by producing specified cuts on a wire. In another embodiment, the proposed elongated device also includes a pusher or pusher wire that is manufactured by producing specified cuts on said tube or said wire.

In an embodiment, the cells in the working portion have an almond shape.

In some embodiments, at least some of the working portion struts can have a width between 30 µm and 60 µm, particularly between 40 µm and 50 µm and more particularly 43 µm or 48 µm.

In some embodiments, at least some of the tapered portion struts can have a width between 60 µm and 155 µm, particularly between 70 µm and 145 µm and more particularly 71 µm or 142 µm.

In some embodiments, the working portion in the expanded configuration can have a length between 20 mm and 50 mm, particularly between 38 mm and 42 mm and more particularly 40 mm.

In some embodiments, the working portion in the compressed configuration can have a length between 10 mm and 60 mm, particularly between 47 mm and 51 mm and more particularly 49 mm.

In some embodiments, the tapered portion in the expanded configuration can have a length between 10 mm and 20 mm, particularly between 13 mm and 17 mm and more particularly 15 mm.

In some embodiments, the tapered portion in the compressed configuration can have a length between 10 mm and 20 mm, particularly between 10 and 14 mm and more particularly 12 mm. In an embodiment, the tapered portion struts have a thickness equal to a thickness of the working portion struts.

In some embodiments, the elongated device also includes radiopaque markers located at a distal end of the working portion of the working element. In other embodiments the radiopaque markers can be also located at other sections of the working portion, for example at the middle and/or at the proximal end. The radiopaque markers particularly have different lengths to avoid entanglement between them or other devices. The radiopaque markers can be made of a Platinum Iridium alloy or of Tantalum.

In an embodiment, the elongated device is made of a metal including Nitinol. In particular, the Nitinol material complies with the ASTM (American Society of Testing and Materials) F2063 (Standard Specification for Wrought Nickel-Titanium Shape Memory Alloys for Medical Devices and Surgical Implants). Nitinol is well known for applications in self-expanding structures. However, other types of metals or even other types of materials can be also used, for example cobalt-chromium alloys or iron alloys such as stainless steel or spring steel.

Embodiments of the present invention provide, according to yet another aspect, an expandable elongated device for extraction of an occlusion from a blood vessel. The elongated device, which can be considered a clot mobilizer device, comprises a working portion having a plurality of crowns (or rows) of cells. Each cell of the working portion includes an open area bordered by two proximal cell struts, two distal cell struts, and two middle cell struts, where: the proximal cell struts each extends distally from a common proximal end to a proximal end of a respective one of the two middle cell struts, the distal cell struts each extends proximally from a common distal end to a distal end of a respective one of the two middle cell struts, each middle cell strut in each crown borders two adjacent cells in that crown. Each of the middle cell struts is adapted and configured to be more flexible than the distal cell strut and proximal cell strut to which it extends. Thus, the cells comprising middle cell struts are configured to transfer a desired flexibility to the working portion. Likewise, the working portion struts are configured to transfer a desired flexibility to the clot mobilizer to be navigated through the vasculature (e.g., the neuroanatomy).

The expandable elongated device comprises a tapered portion extending proximally from a proximal end of the working portion, the tapered portion also having a plurality of tapered portion struts. The tapered portion has a smaller diameter at a proximal end than a diameter of the working portion in an expanded configuration. Moreover, in some embodiments, the tapered portion struts can also have a width greater than a width of the working portion struts, e.g. the distal cell struts, the proximal cell struts and/or the middle cell struts. The tapered portion struts are configured to augment the radial outward force provided by the working portion and to provide pushability to the clot mobilizer to be navigated through the vasculature (e.g., the neuroanatomy) while minimizing buckling and kinking.

The elongated device is configured to have a compressed (or retracted) configuration (or position or state) with a compressed diameter, and an expanded configuration (or position or state) with an expanded diameter. The elongated device has the compressed configuration for example when it is located within a lumen of a narrow catheter or a lumen of a narrow vessel with a small diameter, and has the expanded configuration for example when it is located within a lumen of a vessel with large diameter. The elongated device is configured to self-expand from the compressed position (with a compressed diameter or first diameter) to an expanded position (with an expanded diameter or second diameter). In other words, the elongated device is self-expandable from the compressed configuration to the expanded configuration. Additionally, the elongated device is configured to adapt its shape to the surrounding blood vessel, thus the elongated device can have a first diameter in a narrow vessel with a small diameter and a second diameter in a wide vessel with a large diameter.

In some embodiments, the working portion is configured to expand from a compressed configuration with a first diameter of less than 1.5 mm to an expanded configuration with a second diameter of at least 3.0 mm and to exert an outward radial force between 0.5 N and 3 N at every diameter between and including the first diameter and the second diameter.

In some embodiments, the working portion is configured to exert an outward radial force between 0.5 N and 3 N in a diameter (first diameter) between 1.5 mm and 3.5 mm. In other embodiments, the working portion is configured to exert an outward radial force between 1.0 N and 2.0 N in a diameter (second diameter) between 2.0 mm and 3.0 mm.

In an embodiment, the working portion is configured to exert an outward radial force between 1.4 N and 3.0 N in a first diameter around 1.5 mm; and wherein the working portion is configured to exert an outward radial force between 0.5 N and 1.4 N in a second diameter around 3.5 mm.

In an embodiment, the working portion is configured to exert an outward radial force between 1 N and 2 N in a diameter around 2.0 mm. In an embodiment, the working portion is configured to exert an outward radial force between 1 N and 2 N in a diameter around 3.0 mm.

In an embodiment, the plurality of crowns of cells in the working portion of the elongated device define a tubular-shaped section forming a cylindrically closed structure. In other words, the working portion defines a tubular-shaped section forming a cylindrically closed structure. To that effect, the proposed elongated device can be manufactured by producing specified cuts either on a tube or on a wire. In another embodiment, the elongated device comprises a pusher (or pusher wire) manufactured by producing specified cuts on said wire. A pusher is configured to maneuver the clot mobilizer (e.g. advance the device distally, withdraw the device proximally, and rotate the device about a longitudinal axis from a proximal end of the pusher).

In an embodiment, the clot mobilizer is manufactured by providing a tube; having a longitudinal axis therethrough, providing a stationary source of laser radiation, generating a beam of laser radiation using the source of laser radiation, and cutting a desired pattern into the tube by scanning the beam over a desired region of the tube. In another embodiment, the clot mobilizer is manufactured by providing a wire; having a longitudinal axis therethrough, producing predetermined cuts of the cross section of the wire by means of an ultrashort pulse laser in order to produce a predetermined shape of the stent. A suitable manufacturing method is described e.g., in US10434605B2.

In an embodiment, each middle cell strut in the working portion can comprise at least a hinge portion. Each hinge portion is disposed between the proximal and distal ends of the middle cell strut. In an embodiment, each middle cell strut in the working portion can comprise a hinge portion. In an embodiment, each middle cell strut in the working portion can comprise a hinge portion disposed between its proximal and distal ends. In another embodiment, each middle cell strut in the working portion can comprise two hinge portions.

In an embodiment, the hinge portion can comprise at least a bend. In an embodiment, the hinge portion can comprise a bend. In some embodiments, the bend is disposed in a central portion of the middle cell strut. In other embodiments, the bend is disposed closer to one end of the middle cell strut than to another end of the middle cell strut.

In an embodiment, the hinge portion can comprise two bends.

In some embodiments, the hinge portion can comprise a section of increased curvature.

In some embodiments, the hinge portion and middle cell strut are two parts of a single structure. The hinge portion is therefore integral with the middle cell strut.

In some embodiments, the middle cell struts bordering each cell in the working portion have a width less than a width of the distal cell struts bordering that cell. In some embodiments, the middle cell struts bordering each cell in the working portion have a width less than a width of the proximal cell struts bordering that cell. In some embodiments, the middle cell struts bordering each cell have a width less than a width of the distal cell struts bordering that cell and less than a width of the proximal cell struts bordering that cell.

In some embodiments, the working portion can comprise at least one crown of intermediate cells. In some embodiments, each intermediate cell can comprise an open area bordered by two proximal cell struts intersecting with two distal cell struts.

In some embodiments, the distal cell struts of each intermediate cell in the at least one crown of intermediate cells can comprise the proximal cell struts of cells in an adjacent crown of cells. In some embodiments, the proximal cell struts of each intermediate cell in the at least one crown of intermediate cells can comprise the distal cell struts of cells in an adjacent crown of cells. The crowns of cells adjacent to the at least one crown of intermediate cells can comprise middle cell struts adapted and configured to be more flexible than the distal cell strut and proximal cell strut to which each middle cell strut extends. Therefore, the cells comprising middle cell struts are configured to transfer a desired flexibility to the working portion. And the intermediate cells are configured to transfer strength to the working portion, thus avoiding kinks (or breaks).

In some embodiments, the number of crowns of cells depends on the desired length of the working portion. In some embodiments, the working portion can have at least four crowns of cells. In some embodiments, the working portion can have between four and twelve crowns of cells. Particularly, the working portion can have between six and ten crowns of cells. More in particular, the working portion can have seven, eight or nine crowns of cells.

In some embodiments, each crown of cells in the working portion can have at least three cells. In some embodiments, each crown of cells in the working portion can have at least four cells. In some embodiments, each crown of cells in the working portion can have between four and ten cells, particularly, between four and eight cells. In an embodiment, each crown of cells in the working portion can have four or six cells.

In an embodiment, each crown of cells in the working portion can have four cells. In another embodiment, each crown of cells in the working portion can have six cells.

In an embodiment, the working portion comprises at least four crowns of cells and each crown of cells comprises at least four cells. In another embodiment, the working portion comprises eight crowns of cells and each crown of cells comprises four cells. In another embodiment, the working portion comprises seven crowns of cells and each crown of cells comprises four cells. In another embodiment, the working portion comprises nine crowns of cells and each crown of cells comprises six cells.

In some embodiments, the distal cell struts in the working portion each can have a width greater than or equal to 0.08 mm and less than or equal to 0.13 mm. In other embodiments, the distal cell struts in the working portion each can have a width greater than or equal to 0.10 mm and less than or equal to 0.125 mm. Particularly, the distal cell struts in the working portion each can have a width of 0.10 mm, 0.12 mm, 0.124 mm or 0.125 mm.

In some embodiments, the proximal cell struts in the working portion each have a width greater than or equal to 0.08 mm and less than or equal to 0.13 mm. In other embodiments, the proximal cell struts in the working portion each can have a width greater than or equal to 0.10 mm and less than or equal to 0.125 mm. Particularly, the proximal cell struts in the working portion each can have a width of 0.10 mm, 0.12 mm, 0.124 mm or 0.125 mm.

In some embodiments, the middle cell struts in the working portion each can have a width greater than or equal to 0.05 mm and less than or equal to 0.13 mm. The middle cell struts in the working portion each can have a width greater than or equal to 0.08 mm and less than or equal to 0.125 mm. The middle cell struts in the working portion each can have a width greater than or equal to 0.08 mm and less than or equal to 0.10 mm. The middle cell struts in the working portion each can have a width of 0.055 mm, 0.075 mm 0.08 mm, 0.09 mm 0.10 mm or 0.125 mm.

In some embodiments, the distal cell struts each have a width greater than or equal to 0.10 mm and less than or equal to 0.145 mm, the proximal cell struts each have a width greater than or equal to 0.10 mm and less than or equal to 0.145 mm, and the middle cell struts each have a width greater than or equal to 0.05 mm and less than or equal to 0.10 mm.

In an embodiment, the distal cell struts and the proximal cell struts can have substantially the same width.

In an embodiment, the elongated device further comprises a pusher extending proximally from the proximal connection portion (or first connection portion).

In an embodiment, the proximal connection portion acts as a pusher connector.

In an embodiment, the proximal connection portion can have two proximally extending arms. In an embodiment, the proximal connection portion acts as pusher connector to connect the pusher to the elongated device through the two extending arms. In an embodiment, the proximal connection portion can have a single proximally extending arm. In an embodiment, the proximal connection portion acts as pusher connector to connect the pusher to the elongated device through the single extending arm.

### Brief Description of the Drawings

The previous and other advantages and features will be more fully understood from the following detailed description of embodiments, with reference to the attached figures, which must be considered in an illustrative and non-limiting manner, in which:
Fig. 1 illustrates an elongated device adapted to be advanced distally and withdrawn proximally from a proximal end and rotated about a longitudinal axis from the proximal end, according to an embodiment.
Fig. 2 schematically illustrates the geometry of the cross-sectional view of the first connection portion and the second connection portion, according to an embodiment.
Fig. 3 illustrates a more detailed view of an elongated device adapted to be advanced distally and withdrawn proximally from a proximal end and rotated about a longitudinal axis from the proximal end, according to another embodiment.
Fig. 4 is a flow chart illustrating a method for connecting the working element to the pusher to enable the pusher to maneuver the working element, according to an embodiment.
Fig. 5A and Fig. 5B illustrate two views of the working element of the proposed elongated device, according to some embodiments of the present invention.
Fig. 6A and Fig. 6B illustrate enlarged views of the tapered portion of the working element, according to some embodiments of the present invention.
Fig. 7A, Fig. 7B and Fig. 7C illustrate three views of the working element of the proposed elongated device, according to some embodiments of the present invention.

### Detailed Description of the Invention

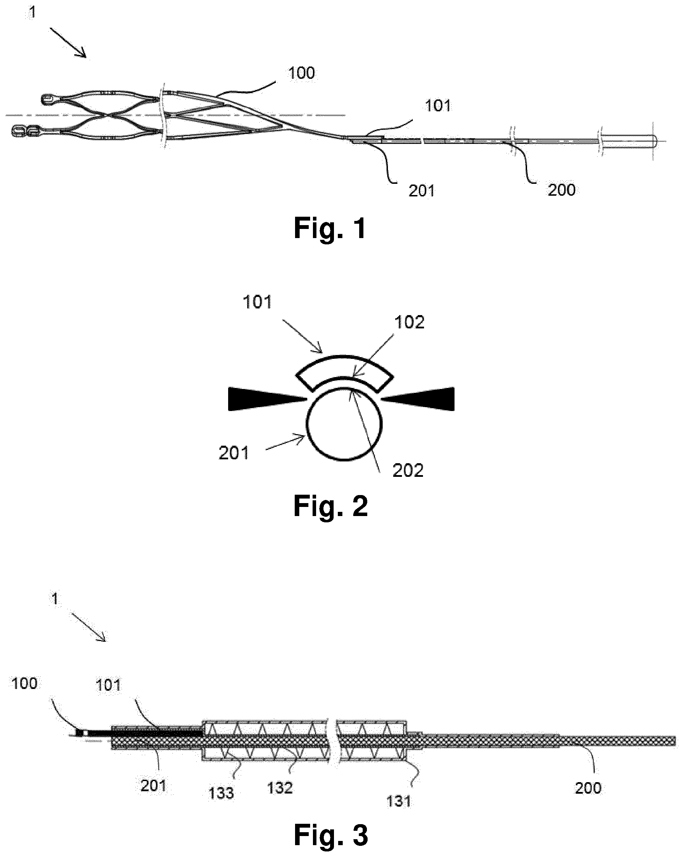

Fig. 1 illustrates an elongated device 1 according to some embodiments of the present invention. As shown in Fig. 1, the proposed elongated device 1 comprises a working element 100 and a pusher 200. The working element 100 can comprise different medical devices, particularly a stent retriever, and includes a first connection portion 101 extending proximally, the first connection portion 101 having a first attachment surface 102 (see Fig. 2). The pusher 200 can comprise a second connection portion 201 extending distally, the second connection portion 201 having a second attachment surface 202 facing and extending longitudinally aligned to the first attachment surface 102 to define an overlapping portion and to define first and second seams extending longitudinally along first and second lateral extents of the overlapping portion.

The elongated device 1 also comprises a first weld attaching the first and second connection portions 101, 201, extending from the first seam toward the second seam, and a second weld attaching the first and second connection portions 101, 201, extending from the second seam toward the first seam.

Fig. 2 illustrates an embodiment of the cross-sectional shape of the first connection portion 101 being a section of an annulus and of the cross-section shape of the second connection portion 201 being a circle. The black triangles show the direction of how the energy, particularly laser energy, is laterally applied to form the first and second welds. In this embodiment, the radius of the second attachment surface 202 is smaller than the radius of the first attachment surface 102.

Particularly, the ratio of the cross-sectional area of the first connection portion 101 perpendicular to the longitudinal axis to the corresponding cross-sectional area of the second connection portion 201 perpendicular to the longitudinal axis is in the range of 1:4 to 2:1; more particularly the ratio is 3:4.

The first weld can comprise multiple welding points along the first seam. Likewise, the second weld can also comprise multiple welding points along the second seam. In an embodiment, the welding points comprise a number between 2 and 12 welding points. In another embodiment, the welding points comprise a number between 2 and 5. The welding points are particularly aligned with each other. In some embodiments, the first weld and the second weld each can additionally comprise a welding seam disposed/arranged over the multiple welding points previously applied and formed by a plurality of consecutive welding points.

In some embodiments, the first weld and the second weld each extend along an entire length of the overlapping portion.

In some embodiments, glue can be added over the first and second welds.

Fig. 3 illustrates another embodiment of the elongated device 1 comprising a jacket 131 or outer jacket, a jacket 132 or inner jacket, and a coil 133 as a radiopaque element extending around the pusher 200. The jacket 132 extends proximally from the overlapping portion over a portion of the pusher 200. The jacket 131 extends around the radiopaque element 133 and part of the overlapping portion. Additionally, a heat shrinkable material can be added to the inner jacket 131 and outer jacket 132.

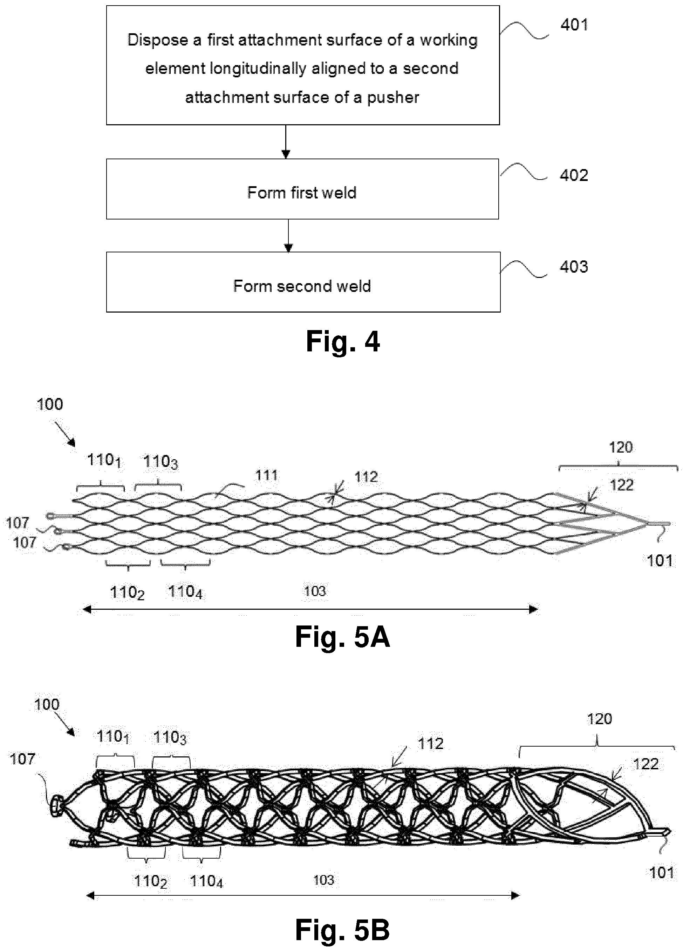

Fig. 4 illustrates a method for associating (i.e. connecting/assembling) the working element 100 to the pusher 200 to enable the pusher 200 to maneuver the working element 100 (i.e. advance the working element 100 distally, withdraw the working element 100 proximally, and rotate the working element 100 about a longitudinal axis from a proximal end of the pusher 200). At step 401 the first attachment surface 102 of the first connection portion 101 is disposed longitudinally aligned to the second attachment surface 202 of the second connection portion 201 to form an overlapping portion and to form first and second seams extending parallel to the longitudinal axis along first and second lateral extents of the overlapping portion. At step 402 a first weld extending from the first seam towards the second seam is formed. At step 403 a second weld extending from the second seam toward the first seam is formed.

In some embodiments, step 402 comprises forming or applying a plurality of welding points (for example between 2 and 12, particularly between 2 and 5) extending from the first seam toward the second seam. Likewise, step 403 comprises forming or applying a plurality of welding points extending from the second seam toward the first seam. In some embodiments, the plurality of welding points are aligned with each other. In some embodiments, steps 402 and 403 also comprise forming a welding seam by consecutively forming or applying a plurality of welding points on top of the previously formed/applied welding points.

To avoid accumulation of heat in specific zones, in some embodiments step 402 further comprises forming a first welding point of the plurality of welding points at a distal portion of the first seam and forming each other welding point of the plurality of welding points at a more proximal location along the first seam than a prior formed welding point of the plurality of welding points, and step 403 further comprises forming a first welding point of the plurality of welding points at a distal portion of the second seam and forming each other welding point of the plurality of welding points at a more proximal location along the second seam than a prior formed welding point of the plurality of welding points. That is, the energy device forming the weld is moved from the distal end of the first (and second) seam to the proximal end of said seam in order to avoid that the first connection portion 101, the second connection portion 102, or both, accumulate the heat which could provoke a breakage thereof.

In certain embodiments, the method includes covering a portion of the pusher 200 proximal to the overlapping portion with a jacket 132 (or inner jacket); placing a radiopaque element 133 at a proximal end of the overlapping portion and extending the radiopaque element 133 around the pusher 200; and covering the radiopaque element 133 with a jacket 131 (or outer jacket) and extending the later at least part of the overlapping portion.

In an embodiment, before inserting the inner jacket 132, the method can further comprise assembling, or placing, the radiopaque element 133 onto a distal end of the pusher 200 and pushing the radiopaque element 133 towards a proximal end of the pusher 200.

In another embodiment, before inserting the outer jacket 131, the method can further comprise placing, or pushing back, the radiopaque element 133 to the distal end of the pusher 200 (or at the proximal end of the overlapping portion), without covering the overlapping portion.

In some embodiments, before inserting the outer jacket 131, the method can further comprise heat shrinking the inner jacket 132 onto the overlapping portion, and after the outer jacket 131 has been inserted, heat shrinking the outer jacket 131 onto the inner jacket 132 and the radiopaque coil 133. The heat shrinking can be made using a suitable shrinkable material, for example a thermoplastic material such as polytetrafluoroethylene (PTFE); fluorinated ethylene propylene (FEP); perfluoroalkoxy alkanes (PFA); ethylene tetrafluoroethylene (ETFE); polyethylene terephthalate (PET) resins, etc.

In other embodiments, the working element 100 can be associated (i.e. connected/assembled) to the pusher 200 by other suitable attachment techniques, such as chemical or thermally bonding, friction or mechanically fitting, crimping, soldering, brazing, or even by using a connector material or member, or combinations thereof. In another embodiment, the working element 100 can be associated to the pusher 200 by crimping a coated or plated band disposed around and crimped to the overlapping portion.

The elongated device 1 can be made of Nitinol, cobalt-chromium alloys, iron alloys such as stainless steel or spring steel, etc.

With reference to Figs. 5A-5B, therein different embodiments of the working element 100 of the elongated device 1 are illustrated. In these embodiments, the working element 100 includes a working portion 103 comprising a plurality of crowns 110, ...110, of almond-shaped cells 111 defining a tubular-shaped section forming a cylindrically closed structure, as can be seen in the 3D representation illustrated in the embodiment of Fig. 5B, and a tapered portion 120 that extends proximally from a proximal end of the working portion 103. For purposes of this disclosure, a "crown" is a closed ring extending circumferentially around the device with alternating longitudinally longer and longitudinally shorter sections. The working portion 103 in a compressed diameter can have a length between 10 mm and 60 mm, and the working portion 103 in an expanded diameter can have a length between 20 mm and 50 mm.

A distal end of each cell 111 in a first crown 110, is contiguous with a proximal end of a corresponding cell 111 in a third crown 110₃, and a distal end of each cell 111 in a second crown 110₂ is contiguous with a proximal end of a corresponding cell 111 in a fourth crown 110₄. The midportions of each cell 111 in each of the crowns each are also contiguous with a midportion of an adjacent cell 111 in such crown.

The working element 100 illustrated in Figs. 5A-5B also include radiopaque markers 107. The radiopaque markers 107 can be incorporated at a distal end of the working portion 103 only (as in Figs. 5A and 5B) or can be also incorporated at other sections of the working portion 103. In some embodiments, the different radiopaque markers 107 have different lengths to avoid entanglement between them or other devices. The radiopaque markers 107 can be made of a Platinum Iridium alloy or of Tantalum.

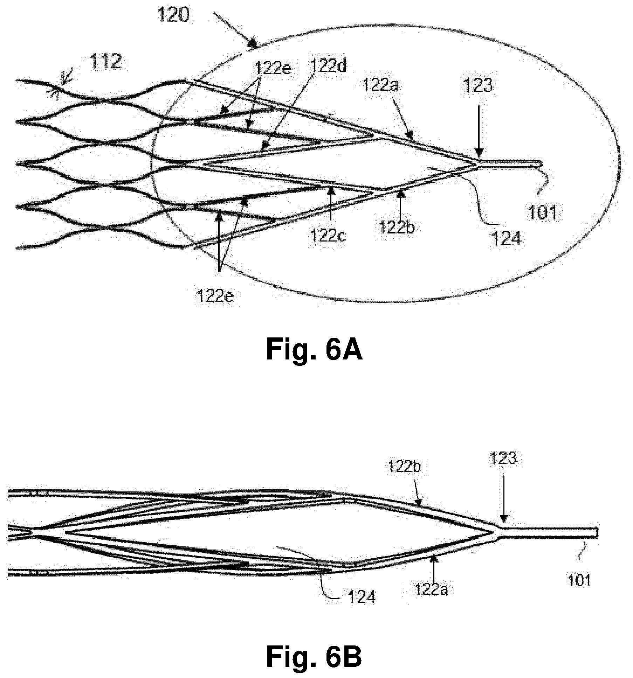

Each one of cells 111 has struts 112. Likewise, the tapered portion 120 also has at least some wide struts 122. Particularly, the wide struts 122 of the tapered portion 120 have a width greater than a width of the working portion struts 112 (see Figs. 6A and 6B for an enlarged illustration thereof).

For example, in the embodiment shown in Figs. 6A and 6B, first 122a and second 122b struts, extending proximally from the proximal end of the working portion 103, converge at a distal end 123 of a proximal connection portion 101 of the working element 100. Third 122c and fourth 122d struts extend distally from first 122a and second 122b struts, respectively, and converge at the proximal end of the working portion 103. First 122a, second 122b, third 122c and fourth 122d struts together define a cell 124 in the tapered portion 120, and these struts 122a-122d are all thicker than the struts 112 in the working portion 103. In the embodiment illustrated in Figs. 6A and 6B, first, second, third and fourth struts 122a-122d are thicker than other struts 122e in the tapered portion 120. These thicker struts 122a-122d help provide greater outward radial force to the working portion 103 from its compressed diameter to its expanded diameter, as discussed below. In some embodiments, because the working portion 103 and the tapered portion 120 of the elongated device 1 is formed from a single hypotube, the wide tapered portion struts 122 have a thickness equal to the thickness of the working portion struts 112, which is predetermined by the thickness of the tube from which it is made. In some embodiments, the working portion struts 112 can have a width between 30 µm and 60 µm, and at least some of the tapered portion struts 122 can have a width between 60 µm and 155 µm.

In some embodiments, the working portion 103 has a compressed diameter of less than 1.5 mm and an expanded diameter of approximately 3.5 mm. In some embodiments, the working portion 103 can exert an outward radial force between 0.75 N and 3 N at every diameter between and including the compressed diameter and the expanded diameter. In an embodiment, the outward radial force exerted by the working portion 103 in a compressed diameter of 1.5 mm is between 1.75 N and 3 N, particularly 2.1 N. In an embodiment, the outward radial force exerted by the working portion 103 in an expanded diameter of 3.5 mm is between 0.75 N and 1.5 N, particularly 1 N.

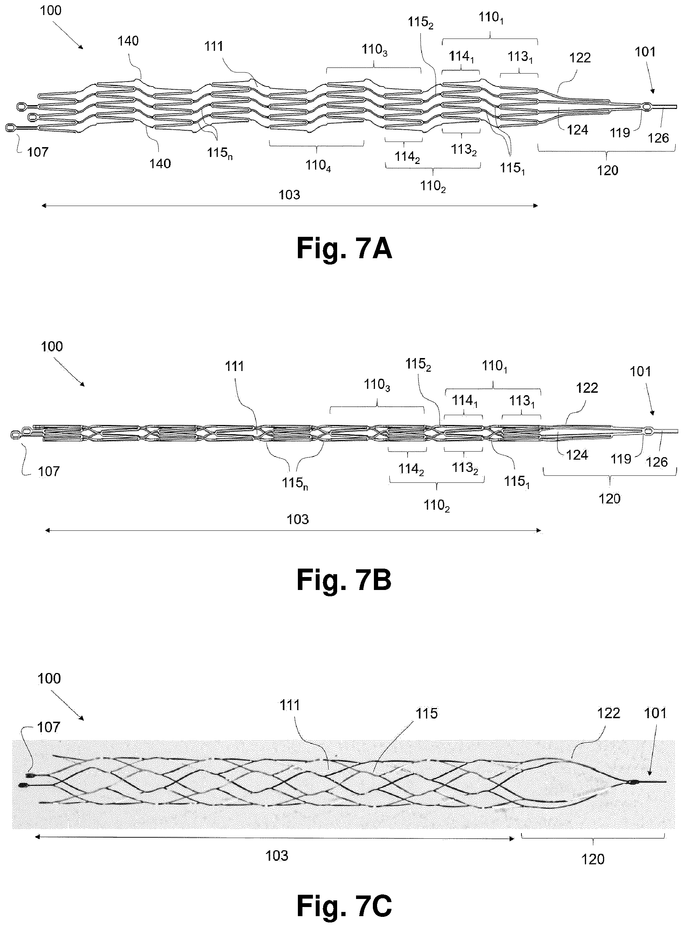

With reference to Figs. 7A-C, therein different embodiments of the proposed working element 100 of the elongated device (or clot mobilizer device) 1 are illustrated. While the various embodiments all have longitudinally extending closed tubes with a tapered proximal end and an open distal end (as shown, e.g., in Fig. 7B), Fig. 7A shows opened and flattened particular embodiments to illustrate two-dimensionally the strut and cell patterns of those embodiments.

The working element 100 includes a working portion 103 comprising a plurality of crowns 110₁ ...110ₙ of cells 111 defining a tubular-shaped section forming a cylindrically closed structure (as can be seen in the 3D representation illustrated in the embodiments of Figs. 7B and 7C). For example, the number of crowns in the plurality of crowns 110₁ ...11 0ₙ can be between 4 and 10, more particularly 6, 7, 8 or 9 (with e.g., n = 1, 2, 3, 4, 5, 6, 7, 8, 9 or 10). As shown in Figs. 7A-C, the working portion 103 of the working element 100 comprises eight crowns of cells. However, the working portion 103, in other embodiments, can comprise seven crowns of cells (not shown) or nine crowns of cells (not shown). For purposes of this disclosure, a "crown" is a closed ring extending circumferentially around the device with alternating longitudinally longer and longitudinally shorter sections. Each crown of cells in the working portion can comprise, for example, four or six cells, and each crown can have components that are shared with one or more adjacent crowns. In some embodiments, the working portion 103 can have a length between 30 mm and 50 mm, for example approximately 40 mm.

Each cell 111ₙ in the crowns of cells 110ₙ in the working portion 103 has an open area bordered by two proximal cell struts, two distal cell struts, and two middle cell struts 115ₙ. For purpose of the disclosure, "n" is used to reference interrelated features; for example, the first crown is referenced as n=1 and its respective adjacent crown as n+1=2, and successively. The distal cell struts of the first crown 110, are disposed in a distal cell strut ring 114₁ at equal distal cell strut positions along a longitudinal axis of the working element 100, and the proximal cell struts of the first crown 110, are disposed in a proximal cell strut ring 113₁ at equal positions along the longitudinal axis of the working element 100. In such crowns of cells, the proximal cell struts each extends distally from a proximal end common to one adjacent proximal cell strut to a distal end common to another adjacent proximal cell strut, where it joins a proximal end of a respective one of the two middle cell struts 115ₙ. Similarly, the distal cell struts each extends proximally from a distal end common to one adjacent distal cell strut to a proximal end common to another distal cell strut, where it joins a distal end of a respective one of the two middle cell struts 115ₙ. Each middle cell strut 115 in each crown borders two adjacent cells 111 in that crown. As shown in Figs 7A-B, the distal strut ring 114ₙ of one crown of cells is the proximal strut ring 113ₙ₊₁ of the adjacent crown of cells, and the distal cell struts of distal strut ring 114ₙ are the proximal cell struts of proximal strut ring 113ₙ₊₁.

The longitudinal and circumferential arrangements of the crowns of cells 110ₙ in the working portion 103 of the working element 100 can affect the behavior of the device in its unexpanded and expanded configurations. For example, providing a circumferential offset between adjacent crowns of cells can facilitate compression of the working element from the expanded configuration into an unexpanded configuration for delivery through a catheter. This circumferential offset of adjacent crowns of cells can also reduce buckling and kinking during advancement of the working element 100 (i.e., clot mobilizer) through the delivery catheter in its unexpanded delivery configuration.

In the embodiment of Figs. 7A-C the common proximal ends of the distal cell strut ring 114₁ of a first crown 110, of cells 111 of the plurality of crowns of cells 110, ...110ₙ (i.e., the proximal ends of the middle cell struts 115₁) are offset from the common distal ends of the proximal cell strut ring 113₁ of the first crown 110, of cells 111 (i.e., the distal ends of the middle cell struts 115₁) in a first circumferential direction, and the common proximal ends of the distal cell strut ring 114₂ of a second crown 110₂ of cells 111 of the plurality of crowns of cells (i.e., the proximal ends of the middle cell struts 115₂) are offset from the common distal ends of the proximal cell strut ring 113₂ of the second crown 110₂ of cells 111 (i.e., the distal ends of the middle cell struts 115₂) in a second circumferential direction opposite to the first circumferential direction. This pattern repeats with subsequent crowns of cells, such that the common distal ends of proximal cell strut rings are alternately offset in the first circumferential direction and in the second circumferential direction in each successive crown of cells. Thus, the crowns are configured one to each other in a zig-zag pattern.

In other embodiments (not shown), the common proximal ends of each distal cell strut ring 114ₙ (i.e., the distal ends of middle cell struts 115ₙ) are circumferentially offset from the common distal ends of the proximal cell strut ring 113ₙ (i.e., the proximal ends of middle cell struts 115ₙ) in the same circumferential direction, such that the middle cell struts 115 between these common ends extend in a direction that is not parallel to the longitudinal axis of the working portion 103. Thus, the crowns are oriented one to each other in a helix pattern.

Likewise, in the embodiment of Figs. 7A-C, each of the middle cell struts 115 is adapted and configured to be more flexible than the distal cell strut and proximal cell strut to which it extends. In this embodiment, the working portion 103 and tapered portion 120 of the working element 100 are cut from a single tube and all elements have the same thickness (i.e., the tube thickness). As shown in the flattened view of Fig. 7A (which shows the pattern to be cut from the solid tube to make the working element 100), the middle cell struts 115 bordering each cell 111 have a width less than a width of the distal cell struts bordering that cell 111 and less than a width of the proximal cell struts bordering that cell 111, thereby making the middle cell struts 115 more flexible than the proximal cell struts and the distal cell struts. For example, the distal cell struts and proximal cell struts can have substantially the same width, e.g., in the range between 0.10 mm and 0.145 mm, particularly 0.10 mm, 0.12 or 0.125 mm, while the middle cell struts 115 can have a width in the range between 0.05 mm 0.13 mm, particularly 0.05, 0.08, 0.09 or 0.10.

To enhance the flexibility of the elongated device 1, each middle cell strut 115 in the working portion 103 also has hinge portions 140 to provide additional flexibility to the middle cell strut. As shown in Fig 7A, the hinge portions 140 are preformed curves or bends in the middle cell strut 115 disposed between the proximal and distal ends of the middle cell strut 115. The hinge portions 140 of this embodiment include two integral bends, one near the common distal end of the proximal cell struts from which the middle cell strut extends and other near the common proximal end of the distal cell struts to which the middle cell strut extends. In other embodiments (not shown), the hinge portions 140 can include a third integral bend in the center of each middle cell strut, much greater than the other two bends. In other embodiments (not shown), the hinge portions 140 in some middle struts 115 are two bends formed closer to the proximal end of the middle cell strut than to the distal end. In other embodiments, each middle cell strut 115 in the working portion 101 can comprise fewer or more hinge portions 140. The hinge portion 140 can be integral with the middle cell strut, as shown e.g., in Figs. 7A, forming, e.g., a living hinge in which the hinge portion 140 and the other portions of the middle cell strut are parts of the same structure. For example, each hinge portion 140 can have an integral section of increased curvature and/or bend. The bend forming the hinge provides added flexibility to the middle cell strut. The additional flexibility of the middle cell struts helps the working element adapt to the shape of the vascular anatomy, e.g., as it is advanced and withdrawn in its unexpanded delivery configuration. The hinges also help the elongated device 1 to adapt its shape to the vascular anatomy into which it is expanded to its expanded configuration to capture a clot. In addition, this feature helps avoid kinking and buckling during advancement by providing multiple bending points along the length of the elongated device 1 to distribute the advancement force when the device encounters resistance to forward movement. In some embodiments (not shown) not all of the crowns of cells have hinge portions.

In other embodiments of the present invention (not shown), one or more crowns of cells of the plurality of crown of cells in the working portion can lack flexible middle cell struts. The working portion 103 can also include at least one crown of intermediate cells, and each intermediate cell can comprise an open area bordered by two proximal cell struts intersecting with two distal cell struts, i.e., omitting the more flexible middle cell struts included in some of the other crowns of cells. The distal cell struts of each intermediate cell in the at least one crown of intermediate cells can comprise the proximal cell struts of cells in an adjacent crown of cells, and the proximal cell struts of each intermediate cell in the at least one crown of intermediate cells can comprise the distal cell struts of cells in an adjacent crown of cells. Therefore, the working portion comprises crowns with flexible middle cells and crowns without flexible middle cells.

The working element 100 of Figs. 7A-C also includes a tapered portion 120 that extends proximally from a proximal end of the working portion 103 to a connection portion 101. The tapered portion 120 has a plurality of struts 122. At least some of the plurality of tapered portion struts 122 border a plurality of tapered portion cells 124. Particularly, the struts 122 of the tapered portion 120 can have a width greater than a width of the working portion struts, e.g., the distal cell struts, the proximal cell struts and/or the middle cell struts 115, thereby making the tapered portion struts stiffer (i.e., less flexible) than the struts of the working portion. The taper of the tapered portion 120 is achieved by the size and number of cells 124 in the tapered portion 120 compared to the sizes and numbers of cells in the working portion 103, with only a single tapered portion cell 124 where struts 122 converge to a connection point 119 distal to a radiopaque marker 107 at a distal end of a proximal connection portion 101.

The tapered portion 120 in the embodiment of Figs. 7A-C has four tapered portion struts 122, two of which extend proximally from the proximal end of the working portion 101 to meet at the connection point 119 distal to the radiopaque marker 107 and a proximally extending arm 126 of a connection portion 121 (arm 126 may connect to a pusher, as described with respect to Figs. 1-4 above). The other two struts 122 extend proximally from the proximal end of the working portion to intersect central portions of the first two struts 122. Together, the four struts define three tapered portion cells 124. Once again, the taper of the tapered portion 120 is achieved by the size and number of cells 124 in the tapered portion 120 compared to the sizes and numbers of cells in the working portion 103. The tapered portion struts help at least the proximal end of the working portion provide a radially outward force due to, e.g., the tapered portion strut angle and/or thickness and the shapes of the cells they define. The tapered portion struts also help with pushability by effectively transmitting a distally-directed advancement force to the working portion of the working element 100.

The proximal connection portion 101 can comprise one or two proximally extending arms 126. Arms 126 may connect to a pusher, as described in other embodiments of the present invention.

The working element 100 can comprise radiopaque markers 107 incorporated at a distal end of the working portion 103 only or can be also incorporated at other sections of the working portion 103. In some embodiments, the different radiopaque markers 107 have different lengths to avoid entanglement between them or other devices. The radiopaque markers 107 can be made of a Platinum Iridium alloy or of Tantalum. The radiopaque markers 107 may be used under fluoroscopy to show the position and degree of expansion of working element 100.

In some embodiments, the working portion 103 has a compressed configuration with a first diameter of less than 1.5 mm and an expanded configuration with a second diameter of at least 3.0 mm. The working portion 103 can exert an outward radial force between 0.5 N and 3.0 N at every diameter between and including the first diameter and the second diameter. In a particular embodiment, the outward radial force exerted by the working portion 103 in a first diameter of 1.5 mm is between 1.4 N and 3.0 N, more particularly 1.4 N, 1.5 N, 2 N or 2.5 N; and the outward radial force exerted by the working portion 103 in a second diameter of 3.5 mm is between 0.5 N and 1.5 N, more particularly 0.6 N, 1 N, 1.4 N or 1.5 N.

In some embodiments, the working portion 103 is configured to exert an outward radial force between 0.5 N and 3 N in a diameter between 1.5 mm and 3.5 mm. In other embodiments, the working portion 103 is configured to exert an outward radial force between 1.0 N and 2.0 N in a diameter between 2.0 mm and 3.0 mm. In a particular embodiment, the working portion 103 is configured to exert an outward radial force between 1 N and 2 N in a diameter around 2.0 mm. In another embodiment, the working portion 103 is configured to exert an outward radial force between 1 N and 2 N in a diameter around 3.0 mm.

The embodiments previously explained can be combined to form a suitable working element 100 such that is configured to exert the proper outward radial force. More examples of working element 100 are described in EP Patent Application No. EP21382165.5.

Following, different examples of the performance of the proposed elongated device 1 are detailed. The examples and drawings are provided herein for illustrative purposes, and without intending to be limiting to the present invention.

### Example 1: Torque resistance test of the attachment (i.e., the connection) between the pusher 200 and the working element 100 of an elongated medical device 1 (in this particular case, a stent retriever).

The purpose of this experimental test was to measure the torque resistance of the attachment between the pusher 200 and the working element 100 of an elongated medical device 1.

Eight stent retrievers were used in this study: Five stent retrievers manufactured by Anaconda Biomed with different configurations (hereinafter Conda devices) and three manufactured by Medtronic (hereinafter Solitaire devices).

### Methods

The tools and equipment used during the procedure are exposed in Table 1.

**Table 1. Tools and equipment used in Example 1.**

| **Tools and Equipment** | **Description** |
|---|---|
| Horizontal Tensile Tester | E7451; Used as a surface to correctly place the fixtures |
| Proximal torque tooling | Fixture used to hold the guide wire torquer and perform and quantify rotational movement |
| Torque Load Cell | Load cell used to register the torque force |
| Torque Load Cell Holder | Fixture used to place the torque load cell |
| Chuck | Element used to hold the stent compressing radially |
| Flatbed Chuck Holder | Fixture used to correctly place the chuck |
| Guide wire torquer | Element used to hold the push wire proximally |
| Hypotube with clamps | Element used to maintain the push wire straight |
| Pin Gauge (0.1645") | Element in which the stent is fitted to avoid its collapse while is being held in the chuck |

### Procedure:

1. The stent retrievers were preconditioned in water at 37°C ± 2°C for at least 2 hours before testing.
2. The 3D printed clamps and proximal torque tooling were set up on the baseboard of the horizontal tensile tester at the following locations and with distal clamp, proximal clamp and proximal torque tooling (placed during setup).
3. The proximal torque tooling and the 3D printed fixture for the proximal torque tooling were placed at the left-hand side of the water bath.
4. The connection portion of the stent retriever was clamped using the guidewire torquer into the jaws of the hand-held torque gauge.
5. The stent retriever was loaded through the hypotube and clamp the distal end of the stent retriever into the Torque Load Cell using a pin gauge.
6. The hand-held torque gauge was set to peak force Nm.
7. The hand-held torque gauge was rotated 360° by allowing the torque gauge holder to click every 90° to aid the operator.
8. The hand-held torque gauge was rotated 360° until a break occurs.
9. When the test was completed, the following details were recorded on a datasheet: Peak Force, Number of Revolutions, Location of Break, Failure Mode.

### Results and conclusion

The following Table 2 shows the results for both, Conda and Solitaire devices:

**Table 2. Comparison torque results between Conda and Solitaire devices.**

| **Nº of revolutions before break** | **Conda** | **Solitaire** |
|---|---|---|
| Mean | 31.2 | 22.0 |
| Minimum | 27.0 | 21.0 |
| Maximum | 34.0 | 23.0 |
| Standard deviation | 3.1 | 1.0 |

The results showed a higher performance of the Conda devices during torque test compared to Solitaire devices. These results are directly related with the safety of the elongated device 1 during the clinical use, therefore, obtaining a higher number of revolutions reduce the possibilities of a premature breakage due to e.g., an incorrect handling of the device. However, the stent retriever devices were not intended for torque during use, so the values obtained for both Conda and Solitaire devices are enough to cover any possible misuse of the elongated device 1.

### Example 2: Tensile test of the attachment (i.e., the connection) between the pusher 200 and the working element 100 of an elongated medical device 1 (in this particular case a stent retriever).

The purpose of this experimental test was to measure the tensile of the connection between the pusher 200 and the working element 100 of an elongated medical device 1.

Thirty-five samples, of them, twenty Conda devices, and fifteen Solitaire devices, were used as stent retrievers to evaluate the tensile strength in the attachment/connection.

### Methods

The tools and equipment used during the procedure are exposed in Table 3.

**Table 3. Tools and equipment used in Example 2.**

| **Tools and Equipment** | **EF- / E-Number** | **Description** |
|---|---|---|
| Zwick Tensile Tester | E11246-01 or equivalent | Equipment with grips with controlled displacement in one axis, connected to Xpert Zwick software |
| 50N Load Cell | Any Calibrated | Load cell installed in the Tensile tester for registering the forces |
| Pneumatic Grips | Record on Use | Grips integrated in the Tensile tester with a opening/closing system by pressurized air |
| Ruler | Any Calibrated | Used to check the distances and |
| Water Bath | Record on Use | Used to precondition the units to 37ºC |
| Thermometer | Any Calibrated | Used to control that the temperature in the preconditioning step is correctly reached |
| Ceramic grips | N/A | Elements integrated into the pneumatic grips that facilitates the correct holding of the devices |

### Procedure:

1. The samples were preconditioned in water at 37°C ± 2°C for at least 2 hours before testing.
2. The parameters were set up on the connected PC through the Zwick test Xpert program (3104236 Rev 01 Clot Mobiliser System Tensile Test.ZP2), as follows: tensile mode, the speed at 4 m/min and grip to grip separation at 10 mm.
3. The configuration of the equipment was checked, and it was ensured that the 50 N load cell was attached, and both pneumatic grips had the ceramic grips attached.
4. A wire of the pusher was placed in the upper grips until the stent bond was positioned in the center point between both sets of pneumatic clamps and the clamps were closed.
5. The force on the tensile tester was set to zero and then the bottom clamp was closed.
6. At this point, the test was started by clicking the button "start".
7. When the test was completed, the following details were recorded on a datasheet: peak tensile force to break bond, location of bond break, failure mode.
8. Finally, when the detail recording was finished, the samples were removed, and the clamps opened.

### Results and conclusion

The following Table 4 shows the results for both, Conda and Solitaire devices:

**Table 4. Comparison tensile results between Conda and Solitaire devices.**

| **Tensile strength (N)** | **Conda** | **Solitaire** |
|---|---|---|
| Mean | 13.4 | 11.6 |
| Minimum | 11.2 | 9.8 |
| Maximum | 17.4 | 13.0 |
| Standard deviation | 1.9 | 1.1 |

The results showed a higher performance of the Conda devices in tensile resistance compared to Solitaire devices. These results are related with the safety of the elongated device 1 during the clinical use, therefore, obtaining higher values of tensile strength reduce the possibilities of a premature breakage of the attachment/connection due to an excessive tension during retrieval.

Unless otherwise indicated, all numbers expressing measurements, conditions, and so forth used in the specification and claims are to be understood as being modified in all instances by the term "about". Accordingly, unless indicated to the contrary, the numerical parameters set forth in this specification and attached claims are approximations that can vary depending upon the desired properties sought to be obtained by the presently disclosed subject matter.

Throughout the description and claims the word "comprise" and its variations such as "comprising" are not intended to exclude other technical features, components, or steps. Additional objects, advantages and features of the invention will become apparent to those skilled in the art upon examination of the description or may be learned by practice of the invention.

The present disclosure and/or some other examples have been described in the above.

According to descriptions above, various alterations may be achieved.

The scope of the present invention is defined in the following set of claims.

## Claims

1. An elongated device for restoring blood flow or removing thrombus, the elongated device being adapted to be advanced distally and withdrawn proximally from a proximal end and rotated about a longitudinal axis from the proximal end, the elongated device comprising:
a working element (100) comprising a first connection portion (101) extending proximally, the first connection portion (101) having a first attachment surface (102);
a pusher (200) comprising a second connection portion (201) extending distally, the second connection portion (201) having a second attachment surface (202) facing and extending longitudinally aligned to the first attachment surface (102) to define an overlapping portion and to define first and second seams extending longitudinally along first and second lateral extents of the overlapping portion;
a first weld attaching the first and second connection portions (101, 201), extending from the first seam toward the second seam; and
a second weld attaching the first and second connection portions (101, 201), extending from the second seam toward the first seam.

2. The device of claim 1, wherein a cross-sectional shape of the first connection portion (101) perpendicular to the longitudinal axis is a section of an annulus and a cross-sectional shape of the second connection portion (201) perpendicular to the longitudinal axis is a circle.

3. The device of any one of claims 1-2, wherein the first weld comprises a plurality of welding points along the first seam and the second weld comprises a plurality of welding points along the second seam.

4. The device of claim 3, wherein the first weld further comprises a plurality of consecutive welding points and the second weld further comprises a plurality of consecutive welding points.

5. The device of any one of claims 1-4, further comprising an inner jacket (132) extending around at least part of the overlapping portion, wherein the inner jacket (132) extends proximally from the overlapping portion around at least part of the pusher (200).

6. The device of any one of claims 1-5, further comprising a radiopaque element (133) disposed at a proximal end of the overlapping portion, wherein the radiopaque element (133) comprises a coil extending around at least part of the pusher (200).

7. The device of any one of claims 5-6, further comprising an outer jacket (131) extending around the radiopaque element (133), wherein the outer jacket (131) extends around at least part of the overlapping portion.

8. A method for connecting a working element (100) of an elongated device for restoring blood flow or removing thrombus to a pusher (200) to enable the pusher (200) to advance the working element (100) distally, withdraw the working element (100) proximally, and rotate the working element (100) about a longitudinal axis from a proximal end of the pusher (200), the working element (100) comprising a first connection portion (101) extending proximally, the first connection portion (101) having a first attachment surface (102), the pusher (200) comprising a second connection portion (201) extending distally, the second connection portion (202) having a second attachment surface (202), the method comprising the following steps:
disposing the first attachment surface (102) longitudinally aligned to the second attachment surface (202) to form an overlapping portion and to form first and second seams extending parallel to the longitudinal axis along first and second lateral extents of the overlapping portion;
forming a first weld extending from the first seam toward the second seam; and
forming a second weld extending from the second seam toward the first seam.

9. The method of claim 8, wherein a cross-sectional shape of the first connection portion (101) perpendicular to the longitudinal axis is a section of an annulus and a cross-sectional shape of the second connection portion (201) perpendicular to the longitudinal axis is a circle.

10. The method of any one of claims 8-9, wherein the step of forming the first weld comprises forming a plurality of welding points extending from the first seam toward the second seam and the step of forming the second weld comprises forming a plurality of welding points extending from the second seam toward the first seam.

11. The method of claim 10, wherein the step of forming the first weld further comprises consecutively forming a plurality of welding points extending from the first seam toward the second seam and the step of forming the second weld further comprises consecutively forming a plurality of welding points extending from the second seam toward the first seam.

12. The method of any one of claims 8-11, wherein the step of forming the first weld further comprises forming a first welding point of the plurality of welding points at a distal portion of the first seam and forming each other welding point of the plurality of welding points at a more proximal location along the first seam than a prior formed welding point of the plurality of welding points, and wherein the step of forming the second weld further comprises forming a first welding point of the plurality of welding points at a distal portion of the second seam and forming each other welding point of the plurality of welding points at a more proximal location along the second seam than a prior formed welding point of the plurality of welding points.

13. The method of any one of claims 8-12, wherein:
the step of forming a first weld comprises forming a first welding point at a proximal end of the first seam and a second welding point between the proximal end and the distal end of the first seam before forming a welding point at any other points along the first seam; and
the step of forming the second weld comprises forming a first welding point at a proximal end of the second seam and a second welding point between the proximal end and the distal end of the second seam before forming a welding point at any other points along the second seam.

14. The method of any one of claims 8-13, wherein the step of forming the first weld comprises directing energy laterally at the first seam toward the second seam and the step of forming the second weld comprises directing energy laterally at the second seam toward the first seam.

15. The method of any one of claims 8-14, further comprising:
covering at least part of the overlapping portion with an inner jacket (132), the inner jacket (132) extending proximally from the overlapping portion around at least part of the pusher (200);
placing a radiopaque element (133) at a proximal end of the overlapping portion, the radiopaque element (133) comprising a coil extending around at least part of the pusher (200); and
covering the radiopaque element (133) with an outer jacket (131), the outer jacket (131) extending around at least part of the overlapping portion.

## Patentansprüche

1. Längliche Vorrichtung zur Wiederherstellung des Blutflusses oder zum Entfernen eines Thrombus, wobei die längliche Vorrichtung dazu angepasst ist, von einem proximalen Ende aus distal vorgeschoben und proximal zurückgezogen und vom proximalen Ende aus um eine Längsachse gedreht zu werden, wobei die längliche Vorrichtung Folgendes umfasst:
ein Arbeitselement (100), umfassend einen ersten Verbindungsabschnitt (101), der sich proximal erstreckt, wobei der erste Verbindungsabschnitt (101) eine erste Befestigungsoberfläche (102) aufweist;
einen Drücker (200), umfassend einen zweiten Verbindungsabschnitt (201), der sich distal erstreckt, wobei der zweite Verbindungsabschnitt (201) eine zweite Befestigungsoberfläche (202) aufweist, die der ersten Befestigungsoberfläche (102) zugewandt ist und sich in Längsrichtung darauf ausgerichtet erstreckt, um einen Überlappungsabschnitt zu definieren und eine erste und eine zweite Naht zu definieren, die sich in Längsrichtung entlang einer ersten und einer zweiten seitlichen Ausdehnung des Überlappungsabschnitts erstrecken;
eine erste Schweißstelle, die den ersten und den zweiten Verbindungsabschnitt (101, 201) verbindet und sich von der ersten Naht in Richtung der zweiten Naht erstreckt; und
eine zweite Schweißstelle, die den ersten und den zweiten Verbindungsabschnitt (101, 201) verbindet und sich von der zweiten Naht in Richtung der ersten Naht erstreckt.

2. Vorrichtung nach Anspruch 1, wobei eine Querschnittsform des ersten Verbindungsabschnitts (101) senkrecht zur Längsachse ein Teil eines Rings ist und eine Querschnittsform des zweiten Verbindungsabschnitts (201) senkrecht zur Längsachse ein Kreis ist.

3. Vorrichtung nach einem der Ansprüche 1-2, wobei die erste Schweißstelle eine Vielzahl von Schweißpunkten entlang der ersten Naht umfasst und die zweite Schweißstelle eine Vielzahl von Schweißpunkten entlang der zweiten Naht umfasst.

4. Vorrichtung nach Anspruch 3, wobei die erste Schweißstelle ferner eine Vielzahl von aufeinanderfolgenden Schweißpunkten umfasst und die zweite Schweißstelle ferner eine Vielzahl von aufeinanderfolgenden Schweißpunkten umfasst.

5. Vorrichtung nach einem der Ansprüche 1-4, ferner umfassend einen Innenmantel (132), der sich mindestens um einen Teil des Überlappungsabschnitts erstreckt, wobei sich der Innenmantel (132) proximal von dem Überlappungsabschnitt um mindestens einen Teil des Drückers (200) erstreckt.

6. Vorrichtung nach einem der Ansprüche 1-5, ferner umfassend ein röntgendichtes Element (133), das an einem proximalen Ende des Überlappungsabschnitts angeordnet ist, wobei das röntgendichte Element (133) eine Spule umfasst, die sich um mindestens einen Teil des Drückers (200) erstreckt.

7. Vorrichtung nach einem der Ansprüche 5-6, ferner umfassend einen Außenmantel (131), der sich um das röntgendichte Element (133) erstreckt, wobei sich der Außenmantel (131) um mindestens einen Teil des Überlappungsabschnitts erstreckt.

8. Verfahren zum Verbinden eines Arbeitselements (100) einer länglichen Vorrichtung zur Wiederherstellung des Blutflusses oder zum Entfernen eines Thrombus mit einem Drücker (200), um es dem Drücker (200) zu ermöglichen, von einem proximalen Ende des Drückers (200) aus das Arbeitselement (100) distal vorzuschieben, das Arbeitselement (100) proximal zurückzuziehen und das Arbeitselement (100) um eine Längsachse zu drehen, wobei das Arbeitselement (100) einen ersten Verbindungsabschnitt (101) umfasst, der sich proximal erstreckt, wobei der erste Verbindungsabschnitt (101) eine erste Befestigungsoberfläche (102) aufweist, wobei der Drücker (200) einen zweiten Verbindungsabschnitt (201) umfasst, der sich distal erstreckt, wobei der zweite Verbindungsabschnitt (202) eine zweite Befestigungsoberfläche (202) aufweist, wobei das Verfahren folgende Schritte umfasst:
Anordnen der ersten Befestigungsoberfläche (102) in Längsrichtung ausgerichtet zu der zweiten Befestigungsoberfläche (202), um einen Überlappungsabschnitt zu bilden und um eine erste und eine zweite Naht zu bilden, die sich parallel zu der Längsachse entlang einer ersten und einer zweiten seitlichen Ausdehnung des Überlappungsabschnitts erstrecken;
Bilden einer ersten Schweißstelle, die sich von der ersten Naht in Richtung der zweiten Naht erstreckt; und Bilden einer zweiten Schweißstelle, die sich von der zweiten Naht in Richtung der ersten Naht erstreckt.

9. Verfahren nach Anspruch 8, wobei eine Querschnittsform des ersten Verbindungsabschnitts (101) senkrecht zur Längsachse ein Teil eines Rings ist und eine Querschnittsform des zweiten Verbindungsabschnitts (201) senkrecht zur Längsachse ein Kreis ist.

10. Verfahren nach einem der Ansprüche 8-9, wobei der Schritt des Bildens der ersten Schweißstelle das Bilden einer Vielzahl von Schweißpunkten umfasst, die sich von der ersten Naht in Richtung der zweiten Naht erstrecken, und der Schritt des Bildens der zweiten Schweißstelle das Bilden einer Vielzahl von Schweißpunkten umfasst, die sich von der zweiten Naht in Richtung der ersten Naht erstrecken.

11. Verfahren nach Anspruch 10, wobei der Schritt des Bildens der ersten Schweißstelle ferner das aufeinanderfolgende Bilden einer Vielzahl von Schweißpunkten umfasst, die sich von der ersten Naht in Richtung der zweiten Naht erstrecken, und der Schritt des Bildens der zweiten Schweißstelle ferner das aufeinanderfolgende Bilden einer Vielzahl von Schweißpunkten umfasst, die sich von der zweiten Naht in Richtung der ersten Naht erstrecken.

12. Verfahren nach einem der Ansprüche 8-11, wobei der Schritt des Bildens der ersten Schweißstelle ferner das Bilden eines ersten Schweißpunktes der Vielzahl von Schweißpunkten an einem distalen Abschnitt der ersten Naht und das Bilden jedes anderen Schweißpunktes der Vielzahl von Schweißpunkten an einer proximaleren Stelle entlang der ersten Naht als ein zuvor gebildeter Schweißpunkt der Vielzahl von Schweißpunkten umfasst, und wobei der Schritt des Bildens der zweiten Schweißstelle ferner das Bilden eines ersten Schweißpunktes der Vielzahl von Schweißpunkten an einem distalen Abschnitt der zweiten Naht und das Bilden jedes anderen Schweißpunktes der Vielzahl von Schweißpunkten an einer proximaleren Stelle entlang der zweiten Naht als ein zuvor gebildeter Schweißpunkt der Vielzahl von Schweißpunkten umfasst.

13. Verfahren nach einem der Ansprüche 8-12, wobei:
der Schritt des Bildens einer ersten Schweißstelle das Bilden eines ersten Schweißpunktes an einem proximalen Ende der ersten Naht und eines zweiten Schweißpunktes zwischen dem proximalen Ende und dem distalen Ende der ersten Naht umfasst, bevor ein Schweißpunkt an beliebigen anderen Punkten entlang der ersten Naht gebildet wird; und
der Schritt des Bildens der zweiten Schweißstelle das Bilden eines ersten Schweißpunktes an einem proximalen Ende der zweiten Naht und eines zweiten Schweißpunktes zwischen dem proximalen Ende und dem distalen Ende der zweiten Naht umfasst, bevor ein Schweißpunkt an beliebigen anderen Punkten entlang der zweiten Naht gebildet wird.

14. Verfahren nach einem der Ansprüche 8-13, wobei der Schritt des Bildens der ersten Schweißstelle das Richten von Energie seitlich an der ersten Naht in Richtung der zweiten Naht umfasst und der Schritt des Bildens der zweiten Schweißstelle das Richten von Energie seitlich an der zweiten Naht in Richtung der ersten Naht umfasst.

15. Verfahren nach einem der Ansprüche 8-14, ferner Folgendes umfassend:
Abdecken mindestens eines Teils des Überlappungsabschnitts mit einem Innenmantel (132), wobei sich der Innenmantel (132) proximal von dem Überlappungsabschnitt um mindestens einen Teil des Drückers (200) erstreckt;
Platzieren eines röntgendichten Elements (133) an einem proximalen Ende des Überlappungsabschnitts, wobei das röntgendichte Element (133) eine Spule umfasst, die sich mindestens um einen Teil des Drückers (200) erstreckt; und
Abdecken des röntgendichten Elements (133) mit einem Außenmantel (131), wobei sich der Außenmantel (131) mindestens um einen Teil des Überlappungsabschnitts erstreckt.

## Revendications

1. Dispositif allongé pour restaurer le flux sanguin ou retirer un thrombus, le dispositif allongé étant adapté pour être avancé distalement et retiré proximalement à partir d'une extrémité proximale et tourné autour d'un axe longitudinal à partir de l'extrémité proximale, le dispositif allongé comprenant :
un élément de travail (100) comprenant une première partie de connexion (101) s'étendant proximalement, la première partie de connexion (101) ayant une première surface de fixation (102) ;
un poussoir (200) comprenant une deuxième partie de connexion (201) s'étendant distalement, la deuxième partie de connexion (201) ayant une deuxième surface de fixation (202) faisant face et s'étendant longitudinalement alignée à la première surface de fixation (102) pour définir une partie de chevauchement et pour définir une première et deuxième coutures s'étendant longitudinalement le long d'une première et deuxième extensions latérales de la partie de chevauchement ;
une première soudure reliant la première et la deuxième parties de connexion (101, 201), s'étendant de la première couture vers la deuxième couture ; et
une deuxième soudure reliant la première et la deuxième parties de connexion (101, 201), s'étendant de la deuxième couture vers la première couture.

2. Dispositif selon la revendication 1, dans lequel la forme en section transversale de la première partie de connexion (101) perpendiculaire à l'axe longitudinal est une section d'un anneau et la forme en section transversale de la deuxième partie de connexion (201) perpendiculaire à l'axe longitudinal est un cercle.

3. Dispositif selon l'une quelconque des revendications 1-2, dans lequel la première soudure comprend une pluralité de points de soudure le long de la première couture et la deuxième soudure comprend une pluralité de points de soudure le long de la deuxième couture.

4. Dispositif selon la revendication 3, dans lequel la première soudure comprend en outre une pluralité de points de soudure consécutifs et la deuxième soudure comprend en outre une pluralité de points de soudure consécutifs.

5. Dispositif selon l'une quelconque des revendications 1-4, comprenant en outre une enveloppe intérieure (132) s'étendant autour d'au moins une partie de la partie de chevauchement, dans lequel l'enveloppe intérieure (132) s'étend proximalement à partir de la partie de chevauchement autour d'au moins une partie du poussoir (200).

6. Dispositif selon l'une quelconque des revendications 1-5, comprenant en outre un élément radio-opaque (133) disposé à une extrémité proximale de la partie de chevauchement, dans lequel l'élément radio-opaque (133) comprend une bobine s'étendant autour d'au moins une partie du poussoir (200).

7. Dispositif selon l'une quelconque des revendications 5-6, comprenant en outre une enveloppe extérieure (131) s'étendant autour de l'élément radio-opaque (133), dans lequel l'enveloppe extérieure (131) s'étend autour d'au moins une partie de la partie de chevauchement.

8. Procédé pour connecter un élément de travail (100) d'un dispositif allongé pour restaurer le flux sanguin ou retirer un thrombus à un poussoir (200) pour permettre au poussoir (200) d'avancer l'élément de travail (100) distalement, de retirer l'élément de travail (100) proximalement, et de faire tourner l'élément de travail (100) autour d'un axe longitudinal à partir d'une extrémité proximale du poussoir (200), l'élément de travail (100) comprend une première partie de connexion (101) s'étendant proximalement, la première partie de connexion (101) ayant une première surface de fixation (102), le poussoir (200) comprenant une deuxième partie de connexion (201) s'étendant distalement, la deuxième partie de connexion (202) ayant une deuxième surface de fixation (202), le procédé comprenant les étapes suivantes :
disposé la première surface de fixation (102) longitudinalement alignée avec la deuxième surface de fixation (202) pour former une partie de chevauchement et pour former une première et une deuxième coutures parallèles à l'axe longitudinal le long d'une première et deuxième extensions latérales de la partie de chevauchement ;
former une première soudure s'étendant de la première couture vers la deuxième couture ; et
former une deuxième soudure s'étendant de la deuxième couture vers la première couture.

9. Procédé selon la revendication 8, dans lequel la forme en section transversale de la première partie de connexion (101) perpendiculaire à l'axe longitudinal est une section d'un anneau et la forme en section transversale de la deuxième partie de connexion (201) perpendiculaire à l'axe longitudinal est un cercle.

10. Procédé selon l'une quelconque des revendications 8-9, dans lequel l'étape de formation de la première soudure comprend la formation d'une pluralité de points de soudure s'étendant de la première couture vers la deuxième couture et l'étape de formation de la deuxième soudure comprend la formation d'une pluralité de points de soudure s'étendant de la deuxième couture vers la première couture.

11. Procédé selon la revendication 10, dans lequel l'étape de formation de la première soudure comprend en outre la formation consécutive d'une pluralité de points de soudure s'étendant de la première couture vers la deuxième couture et l'étape de formation de la deuxième soudure comprend en outre la formation consécutive d'une pluralité de points de soudure s'étendant de la deuxième couture vers la première couture.

12. Procédé selon l'une quelconque des revendications 8-11, dans lequel l'étape de formation de la première soudure comprend en outre la formation d'un premier point de soudure de la pluralité de points de soudure dans une partie distale de la première couture et la formation de chaque autre point de soudure de la pluralité de points de soudure dans un emplacement plus proximal le long de la première couture qu'un point de soudure formé précédemment de la pluralité de points de soudure, et dans lequel l'étape de formation de la deuxième soudure comprend en outre la formation d'un premier point de soudure de la pluralité de points de soudure dans une partie distale de la deuxième couture et la formation de chaque autre point de soudure de la pluralité de points de soudure dans un emplacement plus proximal le long de la deuxième couture qu'un point de soudure formé précédemment de la pluralité de points de soudure,

13. Procédé selon l'une quelconque des revendications 8-12, dans lequel :
l'étape de formation d'une première soudure comprend la formation d'un premier point de soudure dans une extrémité proximale de la première couture et un deuxième point de soudure entre l'extrémité proximale et l'extrémité distale de la première couture avant de former un point de soudure en tout autre point le long de la première couture ; et
l'étape de formation de la deuxième soudure comprend la formation d'un premier point de soudure dans une extrémité proximale de la deuxième couture et un deuxième point de soudure entre l'extrémité proximale et l'extrémité distale de la deuxième couture avant de former un point de soudure en tout autre point le long de la deuxième couture.

14. Procédé selon l'une quelconque des revendications 8-13, dans lequel l'étape de formation de la première soudure comprend diriger de l'énergie latéralement sur la première couture vers la deuxième couture et l'étape de formation de la deuxième soudure comprend diriger de l'énergie latéralement sur la deuxième couture vers la première couture.

15. Procédé selon l'une quelconque des revendications 8-14, comprenant en outre :
recouvrir au moins une partie de la partie de chevauchement avec une enveloppe intérieure (132), l'enveloppe intérieure (132) s'étendant proximalement à partir de la partie de chevauchement autour d'au moins une partie du poussoir (200) ;
placer un élément radio-opaque (133) à une extrémité proximale de la partie de chevauchement, l'élément radio-opaque (133) comprenant une bobine s'étendant autour d'au moins une partie du poussoir (200) ; et
recouvrir l'élément radio-opaque (133) avec une enveloppe extérieure (131), l'enveloppe extérieure (131) s'étendant autour d'au moins une partie de la partie de chevauchement.
